# EUROPEAN PATENT APPLICATION

(11) **EP 4 728 882 A1**
(43) Date of publication of application: **22.04.2026**
(21) Application number: 25209075.8
(22) Date of filing: 16.10.2025
(51) Int. Cl.: A23L 33/135, A61K 35/747, A61P 19/08, A61P 19/10, C12N 1/205, C12N 1/20

(54) **LACTOBACILLUS PARACASEI L-30 STRAIN AND USES THEREOF**

(30) Priority: 18.10.2024 KR 20240143193
(71) Applicant: Neoregen Biotech, Suwon-si, Gyeonggi-do 16614 (KR)
(72) Inventor: SEO, Jeong Min, 06587 Seoul (KR); PARK, Sang Kyu, 03670 Seoul (KR)
(74) Representative: dompatent

(57) **Abstract**

The present invention relates to a novel Lactobacillus paracasei L-30 and uses thereof. More specifically, the present invention is directed to Lactobacillus paracasei L-30 (KCTC16035BP), which has ability to promote osteoblast differentiation or formation, inhibiting osteoclast differentiation or formation, and enhancing immune activity, thereby enabling bone regeneration, and to a pharmaceutical composition including the same for the treatment or prevention of bone diseases.

## Description

### [BACKGROUND OF THE INVENTION]

### 1. Field of the Invention

The present invention relates to a novel Lactobacillus paracasei L-30 strain and uses thereof.

### 2. Description of the Related Art

Osteoporosis is a disease in which the risk of bone fracture increases due to reduced bone density or weakened bone tissue. Osteoporosis is primarily caused by aging, and its incidence is particularly high in women due to hormonal changes after menopause. Bone undergoes continuous remodeling throughout life, in which bone is resorbed and regenerated. In youth, bone regeneration is faster than or similar to the rate of resorption. However, with aging, this balance is disrupted, resulting in a gradual decrease in bone density and mass. Other contributing factors include nutritional deficiencies, such as calcium, phosphate, and vitamin D deficiencies, endocrine disorders, and lack of exercise.

Osteoporosis is a disease that is difficult to cure with short-term medication alone, and it requires long-term drug treatment. Therefore, it is necessary to develop new drugs with novel mechanisms of action and skeletal structures, with minimal toxicity and side effects, and that are effective in preventing and treating osteoporosis. However, bisphosphonates, which account for the majority of existing osteoporosis treatments, cause serious side effects by inhibiting osteoclasts. Accordingly, there is a need to develop natural materials for the prevention and improvement of osteoporosis that are free of side effects, have low toxicity, and are easily administered.

The present inventors have discovered that a novel *Lactobacillus paracasei* strain possesses excellent bone regeneration efficacy and can be used as a composition for the treatment or prevention of bone diseases, thereby completing the present invention.

### [Prior Art Document]

### [Patent Document]

Korean Patent No. 10-2486028

### [SUMMARY OF THE INVENTION]

An object of the present invention is to provide a novel *Lactobacillus paracasei* strain having bone regeneration efficacy.

Another object of the present invention is to provide a pharmaceutical composition including *Lactobacillus paracasei* L-30 for the treatment or prevention of bone diseases.

Still another object of the present invention is to provide a food composition including *Lactobacillus paracasei* L-30 for the improvement of bone diseases.
1. *A Lactobacillus paracasei* L-30 strain deposited under Accession Number KCTC16035BP, having bone regeneration activity.
2. The *Lactobacillus paracasei* L-30 strain according to item 1 above, wherein the strain includes the 16S rRNA sequence of SEQ ID NO: 1.
3. The *Lactobacillus paracasei* L-30 strain according to item 1 above, wherein the strain promotes differentiation or formation of osteoblasts.
4. The *Lactobacillus paracasei* L-30 strain according to item 1 above, wherein the strain inhibits osteoclast differentiation or formation.
5. The *Lactobacillus paracasei* L-30 strain according to item 1 above, wherein the strain promotes immune activity.
6. A pharmaceutical composition for treating or preventing bone diseases, including one or more selected from the group consisting of the *Lactobacillus paracasei* L-30 strain according to any one of items 1 to 5 above, a lysate thereof, a culture thereof, an extract thereof, and a fraction of the extract.
7. The pharmaceutical composition according to item 6 above, wherein the fraction of the extract is a fraction included in the first peak obtained by separating the extract of *Lactobacillus paracasei* L-30 strain by fast protein liquid chromatography (FPLC).
8. The pharmaceutical composition according to item 6 above, wherein the fraction of the extract has a size of 440 to 600 kDa.
9. The pharmaceutical composition according to item 6 above, wherein the bone disease is one or more selected from the group consisting of osteoporosis, localized osteoporosis, osteomalacia, rheumatoid arthritis, degenerative arthritis, osteogenesis imperfecta, osteopenia, bone atrophy, intervertebral disc disease, rickets, fibrous dysplasia, Paget's disease of bone, traumatic fracture, and stress fracture.
10. A food composition for improving bone diseases, including one or more selected from the group consisting of the *Lactobacillus paracasei* L-30 strain according to any one of items 1 to 5 above, a lysate thereof, a culture thereof, an extract thereof, and a fraction of the extract.
11. The food composition according to item 10 above, wherein the fraction of the extract is a fraction included in the first peak obtained by separating the extract of *Lactobacillus paracasei* L-30 strain by fast protein liquid chromatography (FPLC).
12. The food composition according to item 10 above, wherein the fraction of the extract has a size of 440 to 600 kDa.
13. The food composition according to item 10 above, wherein the bone disease is one or more selected from the group consisting of osteoporosis, localized osteoporosis, osteomalacia, rheumatoid arthritis, degenerative arthritis, osteogenesis imperfecta, osteopenia, bone atrophy, intervertebral disc disease, rickets, fibrous dysplasia, Paget's disease of bone, traumatic fracture, and stress fracture.

The *Lactobacillus paracasei* L-30 of the present invention has bone regeneration efficacy.

The *Lactobacillus paracasei* L-30 of the present invention may promote bone formation by activating the differentiation or formation of osteoblasts.

The *Lactobacillus paracasei* L-30 of the present invention may inhibit the differentiation and fusion of osteoclasts, thereby facilitating effective bone regeneration.

The *Lactobacillus paracasei* L-30 of the present invention may promote immune activity.

### [BRIEF DESCRIPTION OF THE DRAWINGS]

The above and other objects, features and other advantages of the present disclosure will be more clearly understood from the following detailed description taken in conjunction with the accompanying drawings, in which:
FIG. 1 is photographs illustrating the comparison of osteogenic differentiation efficacy of various lactic acid bacteria extracts, including the L-30 strain, in human bone marrow stem cells (hBMSCs);
FIG. 2 is a graph illustrating the fractionation of the L-30 extract by size using fast protein liquid chromatography (FPLC);
FIG. 3 is photographs illustrating the comparison of osteogenic differentiation efficacy of different size fractions of the L-30 extract in hBMSCs;
FIG. 4 is photographs illustrating the comparison of osteogenic differentiation efficacy of the L-30 extract and a fraction thereof with extracts of various *Lactobacillus paracasei* strains in hBMSCs;
FIG. 5 is a graph illustrating cell toxicity according to the concentration of the L-30 extract;
FIG. 6 is photographs illustrating the comparison of osteogenic differentiation efficacy according to the concentration and treatment duration of the L-30 extract;
FIG. 7 is graphs and photographs illustrating the expression of osteogenic differentiation markers (mRNA and proteins) following treatment with the L-30 extract;
FIG. 8 is photographs illustrating the comparison of osteogenic differentiation marker expression following treatment with the L-30 extract or a fraction thereof;
FIG. 9 is photographs illustrating the activation of the Wnt pathway following treatment with the L-30 extract;
FIG. 10 is photographs illustrating the effect of p38 or Akt inhibitor treatment on osteogenic differentiation promoted by the L-30 extract;
FIG. 11 is photographs and graphs illustrating the bone regeneration effects of the L-30 extract in mouse cells and tissues;
FIG. 12 is photographs illustrating the comparison of osteoclast differentiation inhibition effects of various lactic acid bacteria extracts, including the L-30 strain;
FIG. 13 is photographs illustrating the comparison of osteoclast differentiation inhibition effects of various *Lactobacillus paracasei* extracts, including the L-30 strain;
FIG. 14 is graphs illustrating the analysis of changes in osteoclast differentiation markers following treatment with the L-30 extract;
FIG. 15 is photographs illustrating the osteoclast differentiation inhibition effects of the peak 1 fraction of the L-30 extract according to concentration;
FIG. 16 is graphs illustrating the analysis of gene expression changes related to osteoclast differentiation following treatment with the L-30 extract;
FIG. 17 is graphs illustrating the expression of osteoclast differentiation receptors following treatment with the L-30 extract;
FIG. 18 is graphs illustrating the expression of osteoclast fusion markers following treatment with the L-30 extract;
FIG. 19 is graphs and photographs illustrating the expression of transcription factors regulating osteoclast differentiation following treatment with the L-30 extract;
FIG. 20 is graphs illustrating the cytotoxicity and ROS expression levels in mouse-derived cells following treatment with the L-30 extract;
FIG. 21 is graphs and photographs illustrating cytokine production following treatment with the L-30 extract; and
FIG. 22 is photographs illustrating the activation of immune-enhancing mechanisms, including the MAPK and NF-κB pathways, following treatment with the L-30 extract.

### [DETAILED DESCRIPTION OF THE INVENTION]

The present invention provides a novel *Lactobacillus paracasei* strain.

The *Lactobacillus paracasei* L-30 strain of the present invention was deposited with the Korean Collection for Type Cultures (KCTC) of the Korea Research Institute of Bioscience and Biotechnology (KRIBB) on September 5, 2024 (Accession No. KCTC 16035BP).

The *Lactobacillus paracasei* L-30 has the 16S rRNA sequence of SEQ ID NO: 1.

The *Lactobacillus paracasei* L-30 possesses bone regeneration efficacy.

The term "bone regeneration" may refer to any process that repairs or rebuilds bone tissue lost due to bone disease or injury, and osteoblasts and osteoclasts may play a key role in the natural bone regeneration process.

The bone regeneration efficacy of the present invention may be due to one or more of the following: the promotion of osteoblast differentiation or formation, or the inhibition of osteoclast differentiation or formation.

The *Lactobacillus paracasei* L-30 strain of the present invention may promote osteoblast differentiation or formation. The strain may increase the expression of osteoblast marker genes or proteins, thereby facilitating osteoblast differentiation and bone formation.

Cells treated with the *Lactobacillus paracasei* L-30 strain of the present invention or an extract thereof may exhibit increased expression of markers promoting osteoblast differentiation. For example, the expression of ALP, RUNX2 or COL1A1 genes related to the promotion of bone differentiation may increase, and the expression of ALP, RUNX2, or COL1A1 proteins may also increase.

Cells treated with the *Lactobacillus paracasei* L-30 strain of the present invention or an extract thereof promote osteoblast differentiation, thereby promoting bone formation, and may be used as a composition for preventing or treating bone diseases.

In one embodiment, when osteogenic differentiation was induced in human adult stem cells, treatment with an extract of the L-30 strain promoted osteogenic differentiation.

In one embodiment, in a group treated with the extract of the L-30 strain, osteogenic differentiation was significantly higher than in groups treated with extracts of other strains.

In one embodiment, osteogenic differentiation was promoted by treating mouse-derived cells or tissues with the L-30 extract after inducing osteogenic differentiation.

The *Lactobacillus paracasei* L-30 strain of the present invention may inhibit osteoclast differentiation or formation. The strain may reduce the expression of osteoclast differentiation marker genes or transcription factors, thereby inhibiting osteoclast differentiation or formation.

In cells treated with the *Lactobacillus paracasei* L-30 strain of the present invention or an extract thereof, osteoclast differentiation may be inhibited, thereby suppressing bone destruction and increasing bone density. In addition, osteoclast fusion may be inhibited, thereby preventing osteoclasts from performing their normal bone resorption function.

Cells treated with the *Lactobacillus paracasei* L-30 strain of the present invention or an extract thereof may exhibit decreased expression of osteoclast differentiation regulators. For example, the expression of NFATc1, which promotes osteoclast differentiation, may be reduced, the gene expression of C/EBPβ and MafB, transcription factors that negatively regulate osteoclast differentiation, may be increased, and the protein expression of p-AKT and p-mTOR may be decreased.

In one embodiment, when cells were treated with an extract of the L-30 strain, representative markers of osteoclast differentiation, including TRAP, Cathepsin K, and MMP-9, were all significantly downregulated at the mRNA level.

Unlike other *Lactobacillus paracasei* strains that exhibit only osteoblast differentiation-promoting activity, the *Lactobacillus paracasei* L-30 strain of the present invention not only promotes osteoblast differentiation but also inhibits osteoclast differentiation. Therefore, it may effectively promote bone regeneration and may be used for the treatment or prevention of bone diseases.

The *Lactobacillus paracasei* L-30 of the present invention has immune activity-promoting efficacy. In cells treated with the L-30 extract, the production of ROS may increase, thereby promoting cytokine production and enhancing immune activity.

The present invention provides a pharmaceutical composition for the treatment or prevention of bone diseases.

The pharmaceutical composition includes one or more selected from the group consisting of the *Lactobacillus paracasei* L-30 strain, a lysate thereof, a culture thereof, an extract thereof, and a fraction of the extract.

The extract of the *Lactobacillus paracasei* L-30 strain of the present invention may include or be prepared from a lysate of a cultured L-30 strain. The extract may be obtained by harvesting and washing the cultured L-30 strain, disrupting the cells by sonication, removing cell walls and other residues by centrifugation, filtering the supernatant, and lyophilizing the filtrate.

The fraction of the extract may be the first peak fraction that appears when separated by fast protein liquid chromatography (FPLC), and the size of the fraction may range from 440 to 600 kDa. The fraction may have superior osteogenic differentiation efficacy compared to the extract of the L-30 strain of the present invention or fractions corresponding to other peaks of the extract.

The extract of the *Lactobacillus paracasei* L-30 strain of the present invention or a fraction thereof may promote bone formation and inhibit osteoclast differentiation or formation, thereby exhibiting effects in the prevention or treatment of bone diseases through enhanced bone regeneration.

The L-30 strain of the present invention, an extract thereof, or a fraction thereof may not exhibit cytotoxicity even when applied to cells at various concentrations.

The term "bone disease" may be one or more bone diseases selected from the group consisting of osteoporosis, localized osteoporosis, osteomalacia, rheumatoid arthritis, degenerative arthritis, osteogenesis imperfecta, osteopenia, bone atrophy, intervertebral disc disease, rickets, fibrous dysplasia, Paget's disease of bone, traumatic fracture, and stress fracture.

As used herein, the term "treatment" refers to any action that improves or beneficially alters a bone disease by administering the pharmaceutical composition of the present invention, and the term "prevention" refers to any action that inhibits or delays the onset of a bone disease.

The term "pharmaceutical composition" may refer to a composition containing the active ingredient alone, or a pharmaceutical composition including one or more pharmaceutically acceptable carriers, excipients, or diluents.

The extract of the *Lactobacillus paracasei* L-30 strain included in the pharmaceutical composition of the present invention may be included at a concentration of 0.1 µg/ml to 100 µg/ml, for example, 0.1 µg/ml to 5 µg/ml, 0.1 µg/ml to 10 µg/ml, 0.5 µg/ml to 20 µg/ml, 0.5 µg/ml to 30 µg/ml, 0.5 µg/ml to 40 µg/ml, 0.5 µg/ml to 50 µg/ml, 0.5 µg/ml to 70 µg/ml, 0.5 µg/ml to 100 µg/ml, or 0.1 µg/ml to 100 µg/ml, but may also exhibit a pharmaceutical effect at concentrations higher than the above range.

The pharmaceutical composition is administered in a pharmaceutically effective amount. The term "pharmaceutically effective amount" refers to an amount sufficient to treat a disease at a reasonable benefit/risk ratio applicable to medical treatment. The effective dose level may be determined depending on factors such as the type and severity of the patient's disease, the activity of the drug, the sensitivity to the drug, the time of administration, the administration route and rate of release, the duration of treatment, concurrent medications, and other factors known in the medical field. The pharmaceutical composition of the present invention may be administered as a single therapeutic agent or in combination with other therapeutic agents. The composition may also be administered sequentially or simultaneously with a conventional therapeutic agent, and may be administered in single or multiple doses. In consideration of the above factors, it is important to administer a minimum amount that can achieve the maximum effect without side effects, and such an amount may be readily determined by those skilled in the art. The effective amount of the pharmaceutical composition may vary depending on the age, gender, and body weight of the patient.

The pharmaceutical composition may be administered orally or parenterally. Parenteral administration may include topical application to the skin, intraperitoneal injection, rectal administration, subcutaneous injection, intravenous injection, intramuscular injection, or intrathoracic injection, but is not limited thereto.

When formulating the composition, it may be prepared using diluents or excipients commonly used in the art, such as fillers, bulking agents, binders, wetting agents, disintegrants, or surfactants.

Solid formulations for oral administration may include tablets, pills, powders, granules, capsules, or troches. Liquid formulations for oral administration may include suspensions, oral solutions, emulsions, or syrups. In addition to water and liquid paraffin, the liquid formulations may further include various excipients, such as wetting agents, sweeteners, flavoring agents, or preservatives.

Formulations for parenteral administration may include sterile aqueous solutions, non-aqueous solutions, suspensions, emulsions, lyophilized preparations, suppositories, or the like.

The present invention provides a food composition for improving bone disease.

The food composition includes one or more selected from the group consisting of the *Lactobacillus paracasei* L-30 strain, a lysate thereof, a culture thereof, an extract thereof, and a fraction of the extract.

The food composition of the present invention, including an extract of the *Lactobacillus paracasei* L-30 strain or a fraction thereof, may promote bone formation and inhibit osteoclast differentiation or formation, thereby improving bone disease through bone formation or bone regeneration.

The food composition may be formulated into a tablet, pill, powder, granule, capsule, or liquid formulation, further including one or more of a carrier, diluent, excipient, or additive. Foods to which the food composition may be added include various products such as powders, granules, tablets, capsules, syrups, beverages, gum, tea, vitamin complexes, and health functional foods.

Additives that may be further included in the food composition include one or more ingredients selected from the group consisting of natural carbohydrates, flavoring agents, nutrients, vitamins, minerals (electrolytes), flavors (synthetic flavors, natural flavors, etc.), coloring agents, fillers (cheese, chocolate, etc.), pectic acid and its salts, alginic acid and its salts, organic acids, protective colloids or thickeners, pH regulators, stabilizers, preservatives, antioxidants, glycerin, alcohol, carbonating agents, and fruit pulp.

When formulating the food composition, it may be manufactured using commonly used diluents or excipients such as fillers, bulking agents, binders, wetting agents, disintegrants, or surfactants.

Hereinafter, the present invention will be described in more detail with examples.

### Example

### Example 1. Isolation of novel Lactobacillus paracasei L-30 strain

Lactobacillus paracasei L-30 strain (KCTC16035BP), obtained from Neorigen Biotech (Gyeonggi-do, Korea), was pre-cultured in MRS medium at 35 to 37 °C for 18 h. The strain was then inoculated at a 1% concentration into 500 mL of MRS broth and further cultured at 35 to 37 °C for 18 h. The cultured L-30 was harvested using a centrifuge (10,000 g for 10 min at 4 °C) and washed twice with PBS. The L-30 was then washed with distilled water to completely remove the MRS broth and PBS. The L-30, resuspended in 20 mL of distilled water, was sonicated on ice for 30 min. To remove cell wall components and other residues, the pellet was centrifuged at 10,000 g for 20 min at 4 °C, and then discarded. The supernatant was filtered (0.2 µm) and frozen at -80 °C. The L-30 extract was then lyophilized to obtain the extract. The obtained L-30 extract was reconstituted with PBS before use. Furthermore, the pH of the L-30 extract was adjusted to 7.0 to identify the characteristics of effective molecules within the L-30 extract.

### Example 2. Comparison of the osteogenic differentiation efficacy of various lactic acid bacteria extracts in hBMSCs.

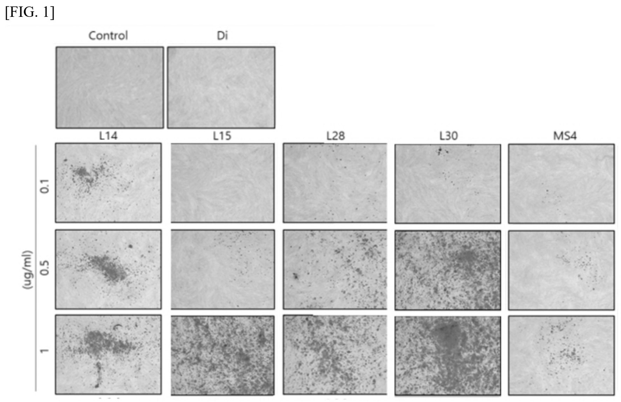

In addition to the L-30 strain of Example 1, extracts of the L14 strain *(Lactobacillus plantarum* L-14), L15 strain (*Enterococcus faecium* L-15), L28 strain (*Lactococcus lactis L-*28), and MS4 strain (*Lactobacillus pentosus* MS-4) were obtained in the same manner as in Example 1. To confirm the osteogenic differentiation effect of the strain of the present invention, the above strains were treated during the induction of osteogenic differentiation in human bone marrow stem cells (hBMSCs). hBMSCs were purchased from PromoCell (Germany) as cells derived from human bone marrow, and the osteogenic differentiation effect was visually confirmed using ARS staining, which visualizes calcium deposits increasing during osteogenic differentiation. The differentiated cells were fixed with 4% paraformaldehyde (PFA) and stained with Alizarin red solution (Sigma-Aldrich). Images were captured using a digital camera (Canon, Tokyo, Japan) and observed using an inverted microscope (EVOS^{™} XL Core Imaging System; Thermo Scientific^{™}, Waltham, MA, USA). As a result, staining was more intense and widespread in the L-30 strain, confirming its superior osteogenic differentiation effect (FIG. 1). This confirmed that the novel strain of the present invention may promote osteoblast differentiation and bone formation.

### Example 3. Size-based separation of extract of Lactobacillus paracasei L-30 strain

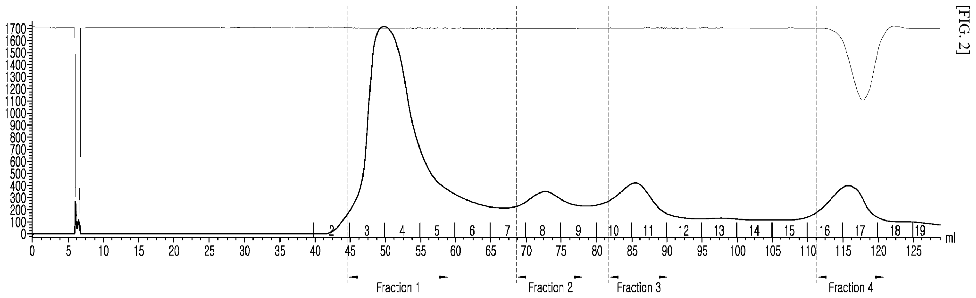

To identify substances effective in osteogenic differentiation from the L-30 extract, fractions were separated by size using fast protein liquid chromatography (FPLC). Specifically, exopolysaccharides (EPS, 30 mg/mL) was separated by size-exclusion chromatography using PBS on a HiLoad^{®} 16/600 Superdex 200 pg column (GE Healthcare) and analyzed with an AKTA fast protein liquid chromatography system (GE Healthcare). As a result, five fraction peaks were separated, as shown in FIG. 2.

### Example 4. Determination of osteogenic differentiation efficacy of size-based fractions of L-30 extract in hBMSCs

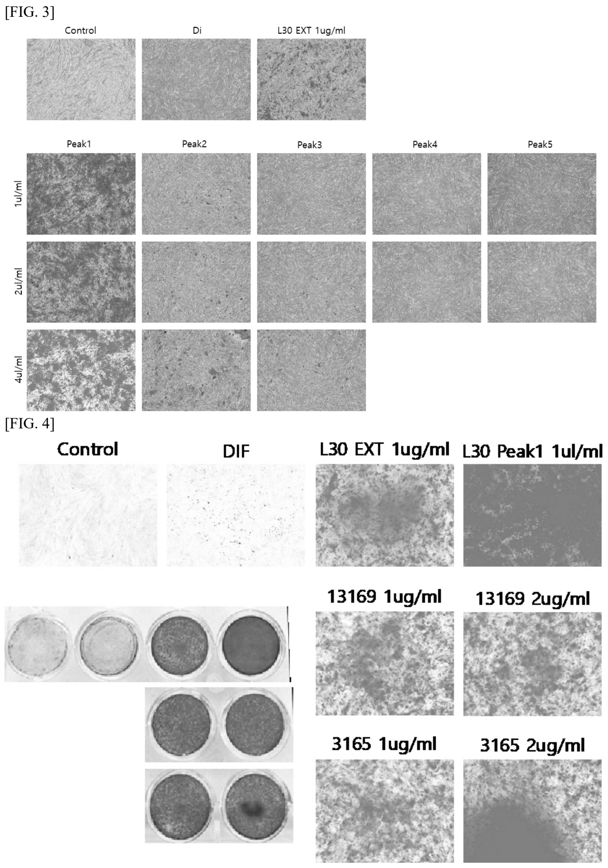

The L-30 extract was separated into five size-based fractions as described in Example 3. The fractions were then applied to differentiating hBMSCs to evaluate the osteogenic differentiation efficacy of each fraction. hBMSCs were seeded at a density of 15,000 cells/cm² in osteogenic differentiation medium composed of α-MEM (Welgene Inc.) containing 10% FBS (Hyclone Laboratories Inc.) and 1% Penicillin-Streptomycin (Gibco Inc.), 100 nM Dexamethasone (Sigma-Aldrich), 10 mM β-glycerol 2-phosphate (Sigma-Aldrich), and 200 µM Ascorbic acid (Sigma-Aldrich) in 24-well plates. The osteogenic differentiation medium was replaced every 2 to 3 days, and osteogenic differentiation was conducted for a total of 21 days. The five fraction peaks separated through FPLC were added to the differentiation medium at concentrations ranging from 1 µl/ml to 4 µl/ml during each medium change, and differentiation was induced for 21 days. The degree of osteogenic differentiation was then observed using the ARS staining technique. As a result, fraction 1 (peak 1) of the L-30 extract exhibited excellent differentiation ability at all tested concentrations and showed superior osteogenic differentiation efficacy compared to the L-30 extract itself (L-30 EXT) (FIG. 3).

### Example 5. Comparison of the osteogenic differentiation efficacy of various Lactobacillus paracasei extracts in hBMSCs

The osteogenic differentiation efficacy of the L-30 extract and its first fraction (peak 1) obtained in the above example was compared with that of extracts from other *Lactobacillus paracasei* strains. L-30 extract (1 µg/ml), peak 1 fraction of the L-30 extract (1 µl/ml), *Lactobacillus paracasei* 13169 (1 µg/ml and 2 µg/ml), and *Lactobacillus paracasei* 3165 (1 µg/ml and 2 µg/ml) were applied to hBMSCs in the same manner as in the above examples. As a result of comparing the degree of osteogenic differentiation, osteogenic differentiation effects were observed in all *Lactobacillus paracasei* strains, including L-30. In particular, the first fraction (peak 1) of the L-30 extract exhibited the highest osteogenic differentiation effect (FIG. 4).

### Example 6. Confirmation of cytotoxicity of the L-30 extract according to the concentration

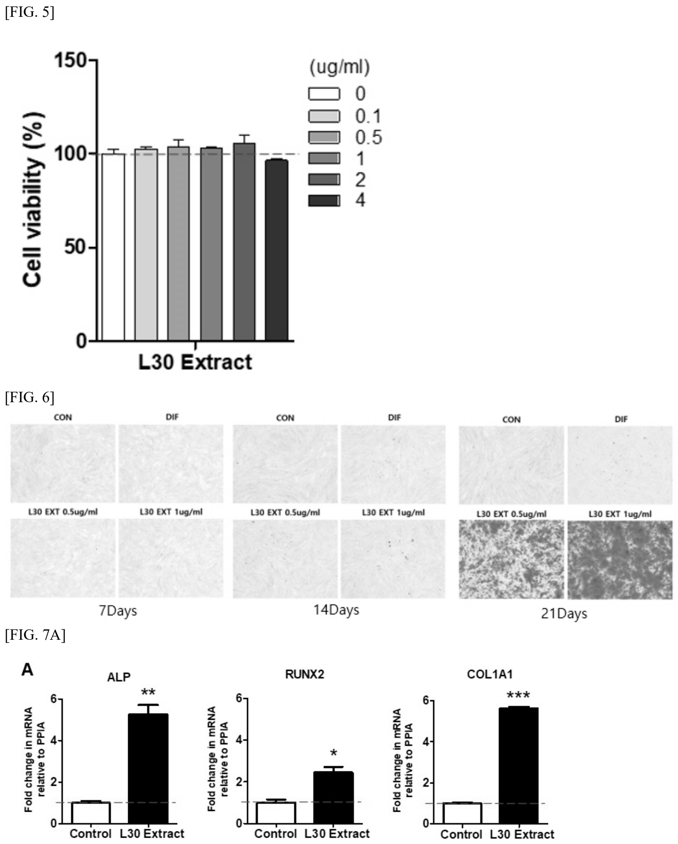

Cytotoxicity (cell viability) tests were performed on the L-30 extract of the above example at different concentrations. The EZ-Cytox kit (Daeil Lab Service, Seoul, Korea), based on the water-soluble tetrazolium salt (WST) method, was used. hBMSCs were seeded at a density of 1.5 × 10⁴ cells per well in 96-well plates. The cells were cultured for 3 days in osteogenic differentiation medium containing various concentrations of the L-30 extract. WST solution was then added to each well, and the mixtures were incubated at 37 °C for 30 min. The absorbance of each well was measured at 450 nm using an Emax Plus Microplate Reader (Molecular Devices, Sunnyvale, CA, USA). As a result, it was confirmed that no cytotoxicity was observed at concentrations within the effective effect range of the extract (FIG. 5).

### Example 7. Comparison of osteogenic differentiation efficacy of L-30 extract according to the concentration and treatment period

The L-30 extract of the above example was applied to osteogenic differentiation medium at varying concentrations. hBMSCs were seeded at a density of 15,000 cells/cm² in osteogenic differentiation medium composed of α-MEM (Welgene Inc.) containing 10% FBS (Hyclone Laboratories Inc.) and 1% Penicillin-Streptomycin (Gibco Inc.), 100 nM Dexamethasone (Sigma-Aldrich), 10 mM β-glycerol 2-phosphate (Sigma-Aldrich), and 200 µM Ascorbic acid (Sigma-Aldrich) in 24-well plates. The medium was replaced every 2 to 3 days, and osteogenic differentiation was conducted for a total of 21 days. Observation using ARS staining confirmed that the L-30 extract effectively induced osteogenic differentiation on day 21 at both 0.5 µg/ml and 1 µg/ml (FIG. 6).

### Example 8. Confirmation of the expression of osteogenesis-related mRNA following treatment with the L-30 extract

The L-30 extract of the above example was applied during the induction of osteogenic differentiation of hBMSCs to examine the expression of markers related to the promotion of osteogenic differentiation. Total RNA was extracted from hBMSCs treated with the L-30 extract using TRIzol^{®} reagent (Invitrogen), and cDNA was synthesized using AccuPower^{®} RT PreMix & Master Mix (BIONEER Co., Korea). qRT-PCR analysis was performed using SYBR Premix Ex Taq^{™} II (Takara, Tokyo, Japan) and a 7500 Real-Time PCR System (Applied Biosystems, Carlsbad, CA, USA). The PCR reaction was carried out at 95 °C for 30 s, followed by 40 amplification cycles of 95 °C for 5 s and 60 °C for 34 s. The comparative CT method was employed to measure expression levels, and Peptidylprolyl isomerase A (PPIA) served as the housekeeping gene for quantification. As a result, ALP, RUNX2, and COL1A1, representative markers of osteogenic differentiation, were all found to be significantly upregulated at the mRNA level compared to the control group (FIG. 7A).

### Example 9. Confirmation of the expression of osteogenic proteins following treatment with the L-30 extract

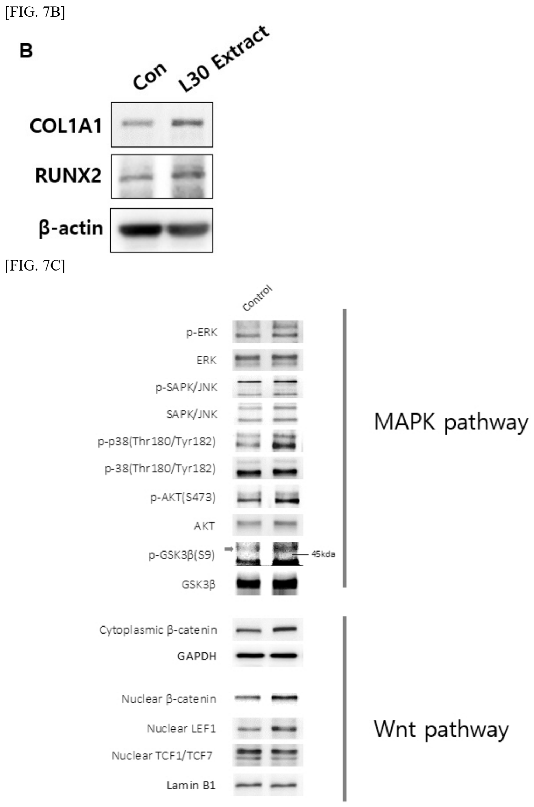

The L-30 extract of the above example was applied during the induction of osteogenic differentiation of hBMSCs. Proteins were then extracted and analyzed by Western blotting to determine the expression of markers related to the promotion of osteogenic differentiation. Cytoplasmic and nuclear proteins were extracted using NE-PER^{™} Nuclear and Cytoplasmic Extraction Reagents (Thermo Scientific^{™}), a proteinase inhibitor (MedChemExpress, Monmouth Junction, NJ, USA), and a phosphatase inhibitor (MedChemExpress). The extracted proteins were separated by electrophoresis on a sodium dodecyl sulfate polyacrylamide gel and subjected to immunoblotting with the following antibodies: runx family transcription factor 2 (Runx2; MBL International, USA), ALPI (CUSABIO, USA), COL1A1 (Cell Signaling Technology), AKT (Cell Signaling Technology), p-AKT (Cell Signaling Technology), extracellular signal-regulated kinase (ERK; Cell Signaling Technology), phospho-ERK (p-ERK; Cell Signaling Technology), c-Jun N-terminal kinase (JNK; Cell Signaling Technology), p-JNK (Cell Signaling Technology), p38 (Cell Signaling Technology), p-p38 (Cell Signaling Technology), p-β-catenin (Thermo Fisher, USA), β-catenin (Cell Signaling Technology), p-GSK3β (Cell Signaling Technology), GSK3β (Cell Signaling Technology), Lamin B1 (Santa Cruz Biotechnology, Dallas, TX, USA), and GAPDH (BioLegend, San Diego, CA, USA). GAPDH served as a housekeeping gene for quantification, and Lamin B1 was used for quantifying nuclear β-catenin. Proteins extracted from hBMSCs treated with the L-30 extract exhibited significantly elevated expression of ALP, RUNX2, and COL1A1 compared to the control group (FIGS. 7B and 7C).

### Example 10. Confirmation of the expression of osteogenesis-related markers following treatment with the L-30 extract or its fraction

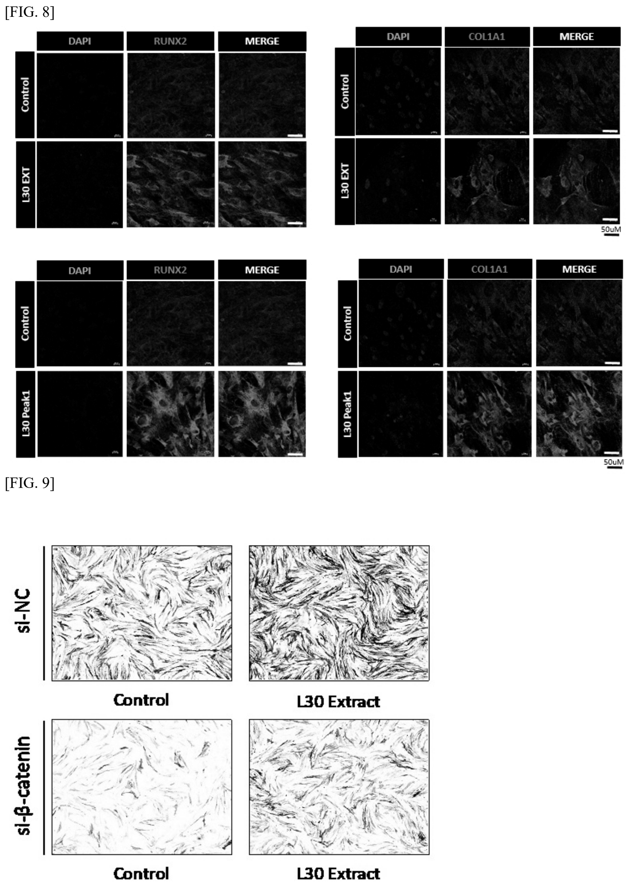

The L-30 extract and its first fraction (peak 1) of the above example were applied during the induction of osteogenic differentiation of hBMSCs. The expression of markers related to the promotion of osteogenic differentiation was examined using fluorescent immunostaining. hBMSCs were fixed with 4% paraformaldehyde and permeabilized with Triton X-100 (in PBS) for 10 min at room temperature. After blocking with 3% BSA (in PBS) for 1 h, the cells were incubated with primary antibodies (in 0.1% BSA). The primary antibodies used were runx family transcription factor 2 (Runx2; MBL International, USA) and COL1A1 (Cell Signaling Technology). The secondary antibody was Goat anti-Mouse IgG (H+L) Cross-Adsorbed Secondary Antibody, Alexa Fluor 594 (Invitrogen). Nuclei were stained with ProLong^{™} Glass Antifade Mountant with NucBlue^{™} Stain (Invitrogen). Staining was observed using a confocal laser scanning microscope (LSM980 with Airyscan2). The results confirmed that both RUNX2 and COL1A1 were strongly expressed in cells treated with the L-30 extract or its peak 1 fraction compared to the control group. In particular, the peak 1 fraction of the L-30 extract exhibited significantly higher expression than the extract itself (FIG. 8).

### Example 11. Analysis of Wnt pathway activation following treatment with the L-30 extract.

si-β-catenin was applied during the induction of osteogenic differentiation to promote the differentiation and growth of osteoblasts while inducing inhibition of the Wnt pathway, a signaling system important for bone tissue maintenance and regeneration. At the same time, L-30 extract was applied to verify, through ALP staining, the compensatory effect of reactivation. β-catenin antisense (Bioneer, Korea) and a negative control were transfected into hBMSCs using Lipofectamine RNAiMAX (Thermo Fisher Scientific) according to the manufacturer's protocol. si-β-catenin and Lipofectamine were incubated separately at room temperature for 5 min, after which they were combined and further incubated for 20 min. The transfection mixture was added to hBMSCs in 24-well plates, and the cells were cultured for 24 h at 37 °C, 5% CO₂, and 95% humidity before use in osteogenic differentiation experiments. ALP staining was performed using a TRACP & ALP double-stain kit (Takara Bio Inc., Japan) in accordance with the manufacturer's instructions. hBMSCs were treated with the L-30 extract for 7 days, fixed with fixative solution for 2 min, washed twice with PBS, and incubated with ALP staining solution for 30 min. After removing the staining solution, the cells were observed under an inverted microscope (EVOS^{™} XL Core Imaging System; Thermo Scientific^{™}, Waltham, MA, USA).

After confirming that si-β-catenin reduced Wnt signaling and thereby decreased osteogenic differentiation efficiency, it was also observed that treatment with the L-30 extract restored osteogenic differentiation reduced by Wnt signaling inhibition. Based on these results, it was confirmed that the osteogenic differentiation-promoting effect of the L-30 extract is related to activation of the Wnt pathway (FIG. 9).

### Example 12. Confirmation of the osteogenic induction by L-30 extract following treatment with p38 or Akt inhibitor

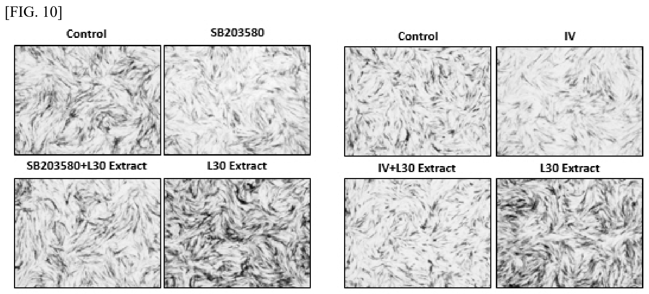

After inhibition of p38 or AKT, the L-30 extract was administered to determine whether the respective pathways could be restored. SB203580 (10 µM) was used as a p38 inhibitor, and AKT inhibitor-IV (10 µM) was used as an AKT inhibitor. The inhibitors were co-administered with 1 µM of the L-30 extract, and the effects of pathway inhibition and restoration were assessed by ALP staining. As a result, it was confirmed that even when the p38 or AKT pathways were inhibited, treatment with the L-30 extract of the present invention restored signaling through the pathways and maintained osteogenic differentiation efficacy (FIG. 10).

### Example 13. Confirmation of bone regeneration effects in mouse-derived cells and tissues

Cells were isolated from mice, cultured, and induced for osteogenic differentiation using the same method and differentiation medium as the hBMSCs described in the above examples. During induction, the L-30 extract was included for treatment. Primary osteoblast precursors were isolated from mouse calvaria. The animal experiments were approved by the Seoul National University Institutional Animal Care and Use Committee (IACUC, approval number SNU-230803-2-2). Calvarias from ICR mice on postnatal day 4 were cultured in 12-well plates in BMSC culture medium in the presence or absence of 10 µg/mL L-30 extract. The medium was replaced every 2 days, and calvarias were harvested on day 7. The calvarias were fixed in 4% PFA for 24 h and decalcified in 14% EDTA for 2 days, then embedded in paraffin wax. The blocks were cut to a depth of 800 µm and sectioned sagittally at a thickness of 10 µm along the midline (Leica Microsystems, Wetzlar, Germany). The sectioned tissues were stained with hematoxylin and eosin (H&E). The separated calvarial sections were digested with 0.25% trypsin and 0.2% collagenase for 30 min at 37°C. Released cells were cultured in α-MEM (Welgene Inc.) supplemented with 10% FBS (Hyclone Laboratories Inc.) in 100-mm plates and maintained at 37°C in a 5% CO₂ incubator. After 3 days, the adherent cells were used as osteoblast precursors. These osteoblast precursors were used for osteogenic differentiation experiments with treatment of the L-30 extract or its peak 1 fraction.

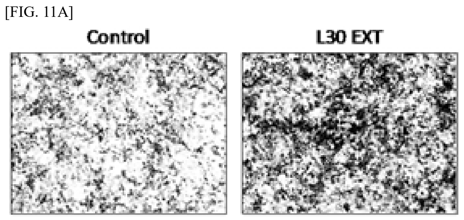

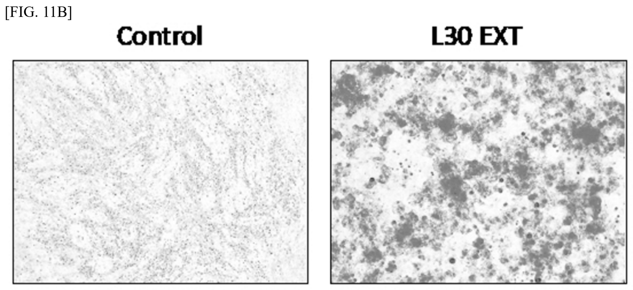

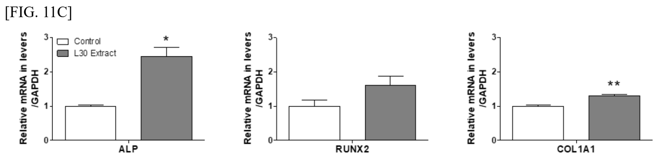

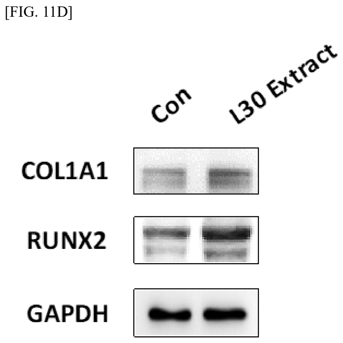

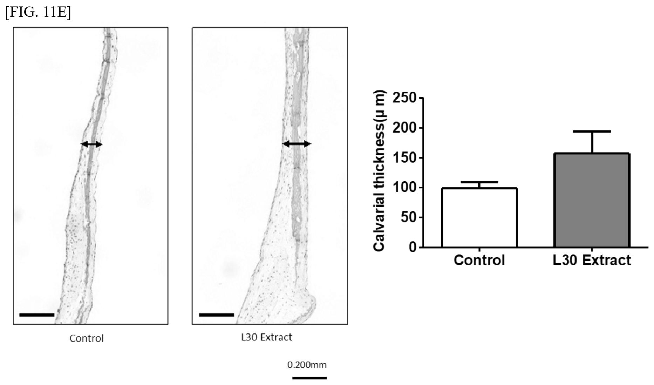

ALP expression was confirmed on day 14 of differentiation (FIG. 11A), and the osteogenic differentiation-promoting efficacy of the L-30 extract was verified on day 21 using the ARS staining method (FIG. 11B). In addition, the increased expression of differentiation-related markers was confirmed by RT-PCR and Western blot (FIGS. 11C and 11D). Furthermore, staining results showed that bone thickness was increased after 7 days of treatment with the L-30 extract, confirming that the extract of the present invention promotes promotes bone regeneration (FIG. 11E).

### Example 14. Comparison of the osteoclast differentiation inhibitory effects of various lactic acid bacteria extracts

Extracts of L14, L15, L28, MS4, and L-30 strains were obtained in the same manner as in Examples 1 and 2 to determine their osteoclast differentiation inhibitory effects. Mouse bone marrow-derived monocytes were isolated, cultured, and differentiated into osteoclasts. Simultaneously, inhibition of osteoclast differentiation was assessed by TRAP staining upon treatment with the above lactic acid bacteria extracts. Monocytes were isolated from the femur and tibia tissues of 6-week-old C57BL6J female mice. The muscle layers surrounding the femur and tibia were removed, disinfected with 70% ethanol, and the joint areas were cut to expose the cells. Bone marrow cells were collected by flushing the bone tissue with α-MEM medium (1% P/S) using a syringe. The collected cells were centrifuged at 2000 rpm for 8 min, and red blood cells were lysed with red blood cell lysis buffer for 10 min. To inactivate the lysis buffer, twice the volume of α-MEM medium (10% FBS, 1% P/S) was added, followed by centrifugation at 2000 rpm for 8 min. After confirming the cell pellet, the supernatant was removed, and the cells were resuspended in fresh α-MEM medium (10% FBS, 1% P/S). Residues were removed using a 70 µm filter. The cells were then treated with 5 ng/ml M-CSF and cultured overnight. The following day, only the monocytes that remained in suspension without adhering to the surface were collected and used for the experiments. To differentiate the monocytes into macrophages, the collected monocytes were treated with 30 ng/ml M-CSF for 3 days. After confirming that the cells had attached to the surface, they were additionally treated with 30 ng/ml M-CSF and 100 ng/ml RANKL for another 7 days to generate multinucleated osteoclasts.

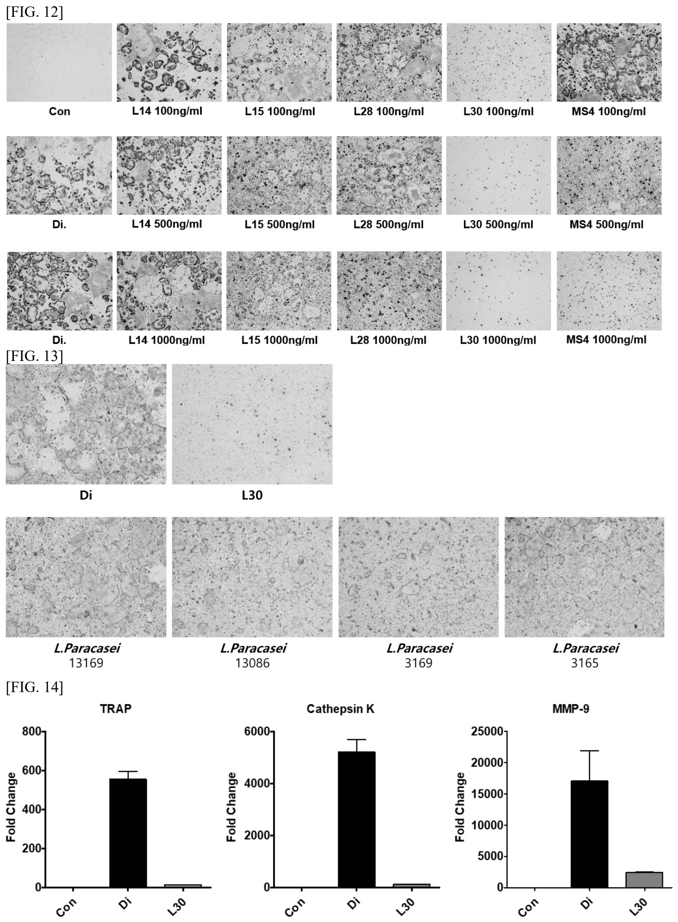

The strains were cultured in MRS medium, washed with PBS to remove residual medium, and sonicated to obtain extracts thereof. Protein concentrations were measured using a BCA assay, and osteoclasts were treated with the extracts together with 30 ng/ml M-CSF and 100 ng/ml RANKL. Osteoclasts treated with each of the above strain extracts were examined for their degree of differentiation by TRAP staining. For staining, cells were washed once with PBS, fixed with 4% PFA for 5 min, and washed again with PBS to remove residual PFA. Sodium tartrate (10% v/v) was added to the TRAP staining solution, mixed, and dispensed onto the cells, followed by incubation at 37°C for 45 min. The stained cells were washed once with PBS and observed under a microscope. As a result, differentiation was inhibited only in osteoclasts treated with the extract of the L-30 strain, in contrast to extracts of other strains (FIG. 12).

Additionally, the osteoclast differentiation inhibitory effects of various *Lactobacillus paracasei* extracts were further evaluated using the same method. Extracts were obtained from *Lactobacillus paracasei* 13169, *Lactobacillus paracasei* 13086, *Lactobacillus paracasei* 3169, *Lactobacillus paracasei* 3165, and the L-30 strain. After treatment with these extracts, osteoclast differentiation was evaluated by TRAP staining. As a result, only the L-30 strain was able to inhibit osteoclast differentiation (FIG. 13), confirming that this inhibitory effect is unique to the L-30 strain of the present invention among *Lactobacillus paracasei* strains.

### Example 15. Confirmation of decreases in osteoclast differentiation markers following treatment with the L-30 extract

The inhibition of osteoclast differentiation by treatment with the L-30 extract was confirmed by monitoring changes in related markers. After completing the induction of differentiation from monocytes into osteoclasts in the same manner as in Example 14, the L-30 extract of the present invention was applied. Quantitative analysis of mRNA expression of osteoclast differentiation markers was performed using the same RT-PCR method as in Example 8. As a result, TRAP, Cathepsin K, and MMP-9, representative markers of osteoclast differentiation, all exhibited significantly decreased expression at the mRNA level compared to the control group (FIG. 14).

### Example 16. Confirmation of the osteoclast differentiation inhibitory effects of L-30 extract fraction according to the concentration

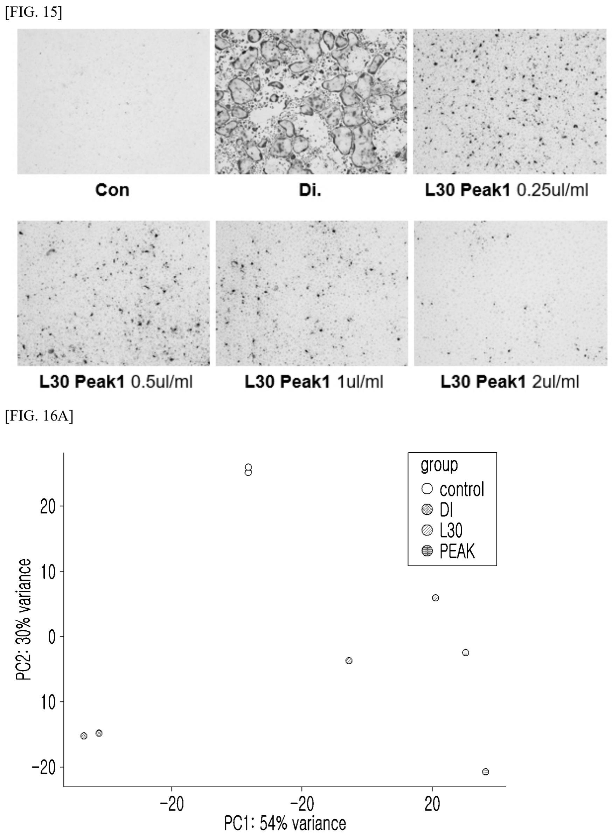

The osteoclast differentiation inhibitory effect of the first fraction (peak 1) of the L-30 extract was evaluated according to its concentration. The peak 1 fraction of the L-30 extract was applied to osteoclasts at concentrations of 0.25 µl/ml, 0.5 µl/ml, 1 µl/ml, and 2 µl/ml, respectively. The degree of differentiation was evaluated by TRAP staining in the same manner as in Example 14. As a result, it was confirmed that the osteoclast differentiation was inhibited at all concentrations of the L-30 fraction (FIG. 15).

### Example 17. Analysis of changes in osteoclast-related gene expression following treatment with the L-30 extract

Gene expression changes in osteoclasts following treatment with the L-30 extract were analyzed. To obtain RNA samples, osteoclasts were seeded at a density of 1 × 10⁶ cells/well in 6-well plates. Osteoclasts were differentiated in the same manner as in the above examples and treated with the L-30 extract. After washing once with PBS, RNA was isolated using Trizol. Chloroform (20% v/v) was added to the Trizol solution containing lysed cells, vortexed, and then centrifuged at 12,000 rpm for 15 min at 4°C to collect only the RNA fraction. The collected RNA was precipitated with isopropanol, pelleted, and then dissolved in RNase-free water. The concentration of the sample was measured using a Nanodrop and RNA Screentape. The obtained RNA sample was subjected to first-strand cDNA synthesis by adding oligo(dT), dNTPs, DTT, and SuperScript III Reverse Transcriptase, followed by incubation in a PCR thermocycler at 55°C for 60 min and 70°C for 15 min. Based on the synthesized first-strand cDNA, second-strand cDNA was synthesized using polymerase buffer, RNase H, and DNA polymerase, followed by performing a reaction at 16°C for 2 h 30 min. The resulting double-stranded DNA was purified 2 to 3 times with magnetic beads and 80% ethanol, and the final product was quantified using a Quantus fluorometer. The DNA was then fragmented with a shearing enzyme (37°C for 7 min and 98°C for 10 min), followed by ligation with adapters A and B at 37°C for 1 h. The ligated product was washed 2 to 3 times with magnetic beads and 80% ethanol. i5/i7 index primers were attached, followed by PCR amplification, and the final products were purified again with magnetic beads and 80% ethanol, and the supernatant was transferred to a new tube.

The resulting RNA library samples were evaluated for concentration and quality using a DNA Tapestation. The samples were pooled to a final concentration of 2 nM using an Illumina P2 300-cycle cartridge, followed by RNA sequencing. The obtained data were subsequently analyzed.

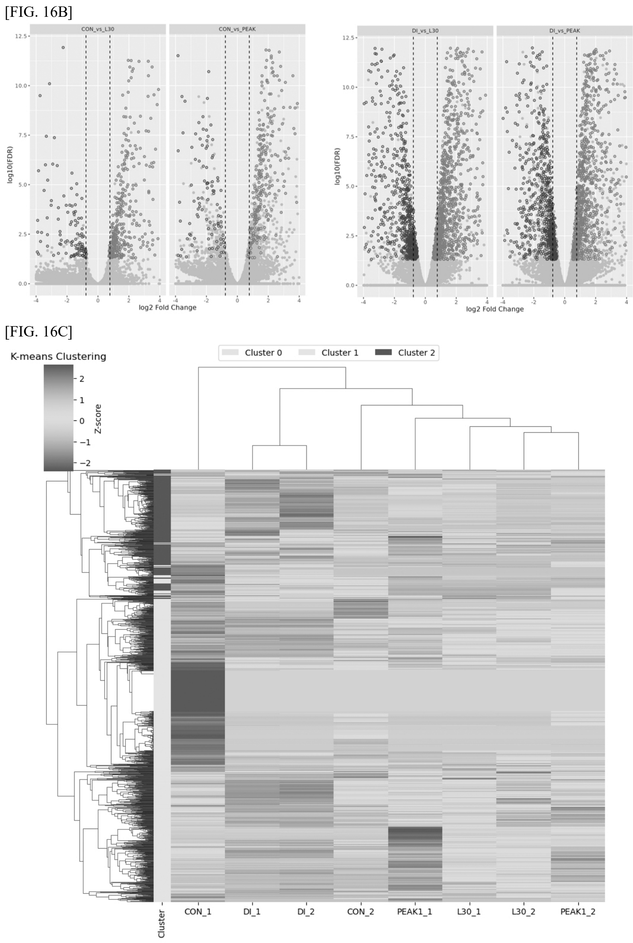

First, PCA plot analysis confirmed that the data for each group (control, L-30 extract (L30), L-30 fraction (peak), and differentiation group (Di)) were appropriately separated, thereby validating the suitability of the samples for analysis (FIG. 16A). Volcano plot analysis further demonstrated that the control group and the L-30 extract or fraction group (FIG. 16B, left) exhibited fewer gene expression changes compared to the differentiation group and the L-30 extract or fraction group (FIG. 16B, right). These results indicate that the L-30 extract or its fraction may suppress osteoclast differentiation.

K-means clustering heatmap analysis revealed that the overall gene expression patterns of the L-30 extract- or fraction-treated groups were more similar to those of the control group (CON_1, CON_2). This indicates that treatment with the L-30 extract or its fraction suppressed the differentiation process, resulting in gene clusters closer to the control group (FIG. 16C).

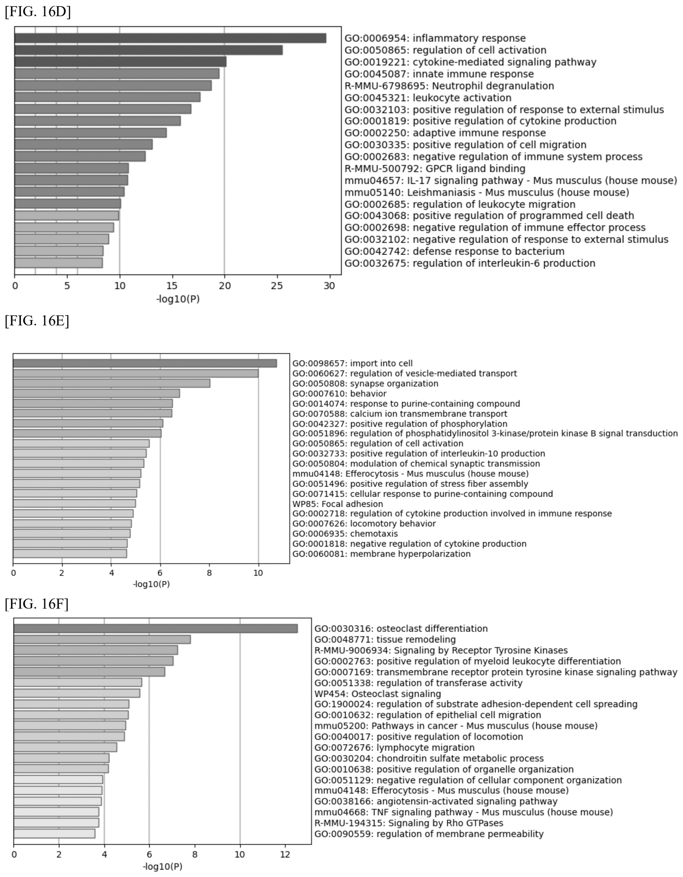

Additionally, GO analysis confirmed that L-30 was highly related to inflammatory, immune, and metabolic mechanisms (FIGS. 16D and 16E), and, among osteoclast-related mechanisms, was particularly strongly related to osteoclast differentiation (FIG. 16F).

### Example 18. Confirmation of the osteoclast differentiation receptor expression following treatment with the L-30 extract.

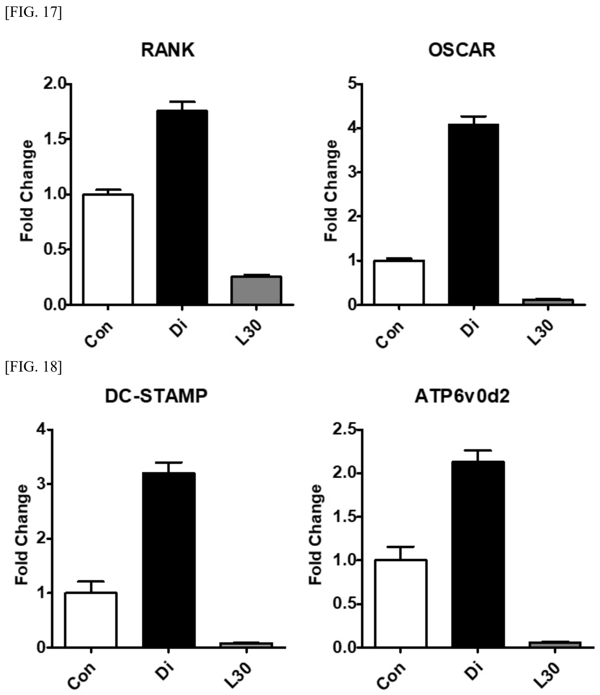

The expression of markers related to differentiation receptors in osteoclasts treated with the L-30 extract was analyzed using RT-PCR. In the same manner as in Example 14, differentiation of monocytes into osteoclasts was induced, followed by treatment with the L-30 extract. The expression of osteoclast differentiation receptors was analyzed using the same RT-PCR method as in Example 8. As a result, the expression of the differentiation receptors RANK and OSCAR were increased during osteoclast differentiation compared to the control group (FIG. 17, Di graph). However, treatment with the L-30 extract markedly reduced the expression of both receptors. These results demonstrate that treatment with the L-30 extract regulates the expression of osteoclast differentiation receptors at the gene level.

### Example 19. Confirmation of the osteoclast fusion marker expression following treatment with the L-30 extract.

For osteoclasts to perform normal bone resorption, the fusion process, in which multiple cells combine, is essential. The expression of DC-STAMP and ATP6v0d2, genes regulating this process, was analyzed. In the same manner as in Example 14, differentiation of monocytes into osteoclasts was induced, followed by treatment with the L-30 extract. In comparison with osteoclasts (Di) not treated with the L-30 extract, no large multinucleated cells were observed, even by gross observation. The expression of osteoclast fusion-related genes was examined using the same RT-PCR method as in Example 8. As a result, the expression of both DC-STAMP and ATP6v0d2 was significantly decreased in the L-30 extract-treated group compared to the Di group. These findings demonstrate that the L-30 extract inhibits osteoclast fusion (FIG. 18).

### Example 20. Confirmation of the expression of transcription factors regulating osteoclast differentiation following treatment with the L-30 extract

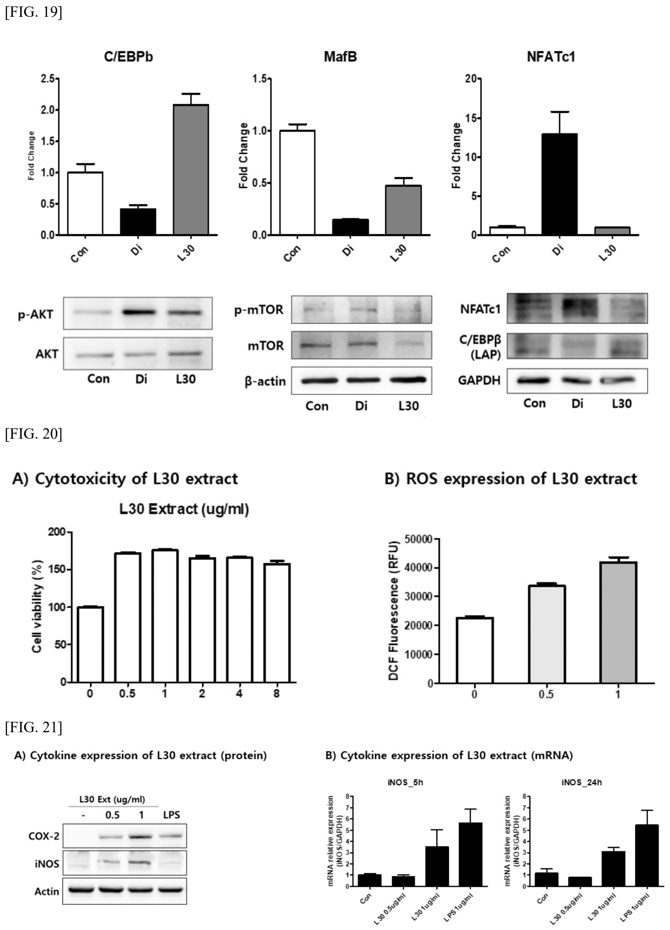

Differentiation of monocytes into osteoclasts was induced in the same manner as in Example 14, followed by treatment with the L-30 extract. Using the same RT-PCR and Western blot methods as in Examples 8 and 9, the gene and protein expression levels of transcription factors regulating osteoclast differentiation were analyzed. NFATc1, which promotes osteoclast differentiation, was decreased at both the gene and protein levels upon treatment with the L-30 extract. In contrast, C/EBPβ and MafB, transcription factors that negatively regulate NFATc1, exhibited increased gene expression, whereas the protein expression levels of p-AKT and p-mTOR were decreased. These findings demonstrate that treatment with the L-30 extract inhibits osteoclast differentiation by suppressing the expression of NFATc1, a key regulator of osteoclasts (FIG. 19).

### Example 21. Confirmation of cytotoxicity and ROS expression levels following treatment with the L-30 extract

Cytotoxicity and reactive oxygen species (ROS) levels were evaluated in mouse-derived cells following treatment with the L-30 extract. Mouse RAW 264.7 macrophages were purchased from the American Type Culture Collection (ATCC) and cultured in DMEM supplemented with 10% fetal bovine serum (FBS) and 1% penicillin/streptomycin. Cells were maintained in an incubator at 37°C and 5% CO₂, and subcultured at 70 to 80% confluency.

RAW 264.7 cells were seeded at a density of 5.0 × 10³ cells/well in 96-well plates, and then treated with the L-30 extract. Cytotoxicity was evaluated after 24 h in the same manner as in Example 6, and no cytotoxicity was observed in cells treated with the L-30 extract (FIG. 20A).

Meanwhile, ROS production in RAW 264.7 cells was assessed using a cell-based ROS detection assay kit (DCFDA, Cayman). RAW 264.7 cells were seeded at a density of 1.0 × 10⁴ cells/well in 96-well culture plates. On the following day, the cells were treated with medium containing the L-30 extract for 24 h. Thereafter, ROS staining buffer (10 µM) was added, and the cells were incubated for 90 min at 37°C under light-protected conditions. Fluorescence was measured using a fluorescence plate reader (TECAN) with an excitation wavelength of 500 nm and an emission wavelength of 550 nm. As a result, treatment with the L-30 extract increased ROS production (FIG. 20B). Since ROS can promote macrophage activation by inducing cytokine production and activating the MAPK and NF-κB pathways, these findings indicate that treatment with the L-30 extract of the present invention may enhance immune function.

### Example 22. Confirmation of the effect of L-30 extract treatment on cytokine production

The expression of COX-2 and iNOS in activated macrophages was examined. RAW 264.7 cells were treated with the L-30 extract, and Western blot analysis was performed after 48 h. In addition, iNOS expression was further analyzed by RT-PCR at 5 h and 24 h after treatment. As a result, treatment with the L-30 extract led to increased expression levels of both COX-2 and iNOS (FIG. 21).

### Example 23. Confirmation of the activation of pathways related to immune enhancement following treatment with the L-30 extract

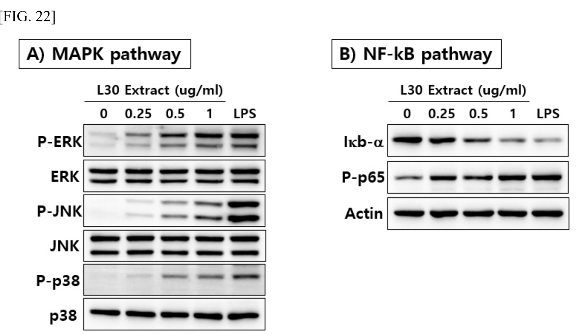

To determine whether L-30 treatment activates mechanisms related to immune enhancement, RAW 264.7 cells were treated with the L-30 extract. Markers related to MAPK and NF-κB pathways were examined 24 h after treatment using Western blot analysis. Activation of NF-κB and MAPKs (p38, ERK, and JNK), which are key pathways involved in the innate immune response, was observed. These results confirmed that L-30 treatment may activate macrophages and thereby enhance immune function (FIG. 22).

### [Accession Number]

Name of Depositary Authority: Korea Research Institute of Bioscience and Biotechnology (KRIBB), Korean Collection for Type Cultures (KCTC)
Accession No.: KCTC16035BP
Accession Date: 20240905

### [Sequence Listing]

Sequence listing electronic file attached
(C:\KipoNet\NKEditor\Data\Hlz\24P08033_sequence listing.xml)

## Claims

1. A *Lactobacillus paracasei* L-30 strain deposited under Accession Number KCTC16035BP, having bone regeneration activity.

2. The *Lactobacillus paracasei* L-30 strain according to claim 1, wherein the strain comprises the 16S rRNA sequence of SEQ ID NO: 1.

3. The *Lactobacillus paracasei* L-30 strain according to claim 1, wherein the strain promotes differentiation or formation of osteoblasts.

4. The *Lactobacillus paracasei* L-30 strain according to claim 1, wherein the strain inhibits osteoclast differentiation or formation.

5. The *Lactobacillus paracasei* L-30 strain according to claim 1, wherein the strain promotes immune activity.

6. A pharmaceutical composition for treating or preventing bone diseases, comprising one or more selected from the group consisting of the *Lactobacillus paracasei* L-30 strain according to any one of claims 1 to 5, a lysate thereof, a culture thereof, an extract thereof, and a fraction of the extract.

7. The pharmaceutical composition according to claim 6, wherein the fraction of the extract is a fraction included in the first peak obtained by separating the extract of *Lactobacillus paracasei* L-30 strain by fast protein liquid chromatography (FPLC).

8. The pharmaceutical composition according to claim 6, wherein the fraction of the extract has a size of 440 to 600 kDa.

9. The pharmaceutical composition according to claim 6, wherein the bone disease is one or more selected from the group consisting of osteoporosis, localized osteoporosis, osteomalacia, rheumatoid arthritis, degenerative arthritis, osteogenesis imperfecta, osteopenia, bone atrophy, intervertebral disc disease, rickets, fibrous dysplasia, Paget's disease of bone, traumatic fracture, and stress fracture.

10. A food composition for improving bone diseases, comprising one or more selected from the group consisting of the *Lactobacillus paracasei* L-30 strain according to any one of claims 1 to 5, a lysate thereof, a culture thereof, an extract thereof, and a fraction of the extract.

11. The food composition according to claim 10, wherein the fraction of the extract is a fraction included in the first peak obtained by separating the extract of *Lactobacillus paracasei* L-30 strain by fast protein liquid chromatography (FPLC).

12. The food composition according to claim 10, wherein the fraction of the extract has a size of 440 to 600 kDa.

13. The food composition according to claim 10, wherein the bone disease is one or more selected from the group consisting of osteoporosis, localized osteoporosis, osteomalacia, rheumatoid arthritis, degenerative arthritis, osteogenesis imperfecta, osteopenia, bone atrophy, intervertebral disc disease, rickets, fibrous dysplasia, Paget's disease of bone, traumatic fracture, and stress fracture.
